(19) Europäisches Patentamt
European Patent Office
Office européen des brevets

(11) EP 4 603 459 A1

(12) EUROPEAN PATENT APPLICATION
published in accordance with Art. 153(4) EPC

(43) Date of publication:
20.08.2025 Bulletin 2025/34

(21) Application number: 23877368.3

(22) Date of filing: 13.10.2023

(51) International Patent Classification (IPC):
*C01G 9/02 (2006.01)* *C09D 17/00 (2006.01)*
*A61Q 1/00 (2006.01)* *A61K 8/27 (2006.01)*
*C09C 3/10 (2006.01)*

(52) Cooperative Patent Classification (CPC):
A61K 8/27; A61Q 1/00; C01G 9/02; C09C 3/10; C09D 17/00

(86) International application number:
PCT/JP2023/037244

(87) International publication number:
WO 2024/080367 (18.04.2024 Gazette 2024/16)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 13.10.2022 JP 2022164568

(71) Applicant: Sumitomo Osaka Cement Co., Ltd.
Tokyo, 105-8641 (JP)

(72) Inventors:
- MATSUSHITA Hirokazu
Tokyo 105-8641 (JP)
- ITO Tomomi
Tokyo 105-8641 (JP)

(74) Representative: Becker, Eberhard
Becker Kurig & Partner
Patentanwälte mbB
Bavariastraße 7
80336 München (DE)

# (54) POLYSILOXANE-COATED METAL OXIDE PARTICLES, DISPERSION LIQUID, COMPOSITION, COSMETIC, AND METHOD FOR PRODUCING POLYSILOXANE-COATED METAL OXIDE PARTICLES

(57) The present invention also addresses the problem of providing a dispersion liquid, a composition and a cosmetic each containing the dimethylpolysiloxane-coated metal oxide particles. The present invention also addresses the problem of providing a method for producing the dimethylpolysiloxane-coated metal oxide particles. The present invention relates to: polysiloxane-coated metal oxide particles, in which the surfaces of metal oxide particles are coated with dimethylpolysiloxane, and which have a hydroxyl group detection ratio of 10% or less; and a method for producing polysiloxane-coated metal oxide particles, the method comprising a surface treatment step for mixing metal oxide particles with dimethylpolysiloxane having a kinematic viscosity of 500 $mm^2/s$ to 4000 $mm^2/s$ inclusive at 25°C, without using a solvent, to coat the surfaces of the metal oxide particles with the dimethylpolysiloxane and a step for subjecting the dimethylpolysiloxane-coated metal oxide particles to a heat treatment at 250°C to 380°C inclusive.

EP 4 603 459 A1

## Description

TECHNICAL FIELD

The present invention relates to polysiloxane-coated metal oxide particles, a dispersion liquid, a composition, a cosmetic, and a method for producing polysiloxane-coated metal oxide particles.
This application claims priority based on Japanese Patent Application No. 2022-164568 filed on October 13, 2022, the content of which is incorporated herein by reference.

BACKGROUND ART

Metal oxide particles formed of titanium oxide, zinc oxide, zirconium oxide, or the like have various characteristics. For example, the characteristics are optical characteristics, mechanical characteristics, thermal characteristics, electrical characteristics, and the like.
A hydroxyl group is present on the surface of the metal oxide particles. Therefore, it is difficult to directly mix the metal oxide particles with a hydrophobic material.
Therefore, the metal oxide particles are used by being mixed with an oil-based cosmetic, a coating material containing an organic solvent, or a composition containing a hydrophobic resin after being subjected to a surface treatment with a hydrophobic surface treatment agent.
For example, zinc oxide particles are used in cosmetics such as sunscreen and foundation. In a case where the zinc oxide particles are applied to a cosmetic, the zinc oxide particles are subjected to the surface treatment to match the surface state of the zinc oxide particles with the properties of the cosmetic. As one of such surface treatment agents for zinc oxide particles, silicone oil such as dimethylpolysiloxane or methylhydrogenpolysiloxane is used (for example, see Patent Literature No. 1).
For example, Patent Literature No. 2 proposes a powder for collecting fingerprints in which a pigment such as titanium oxide, aluminum oxide, or zinc oxide is subjected to a surface treatment with dimethylpolysiloxane or the like.

Citation List

Patent Literature

Patent Literature No. 1: Japanese Laid-open Patent Publication No. 2018-12679
Patent Literature No. 2: Japanese Laid-open Patent Publication No. 1998-155774 (H10-155774)

SUMMARY OF INVENTION

Technical Problem

However, the metal oxide particles with surfaces coated with dimethylpolysiloxane have not exhibited sufficient hydrophobicity. Therefore, it was difficult to mix the metal oxide particles with surfaces coated with dimethylpolysiloxane with oil-based materials and materials such as resins, which have high hydrophobicity. Therefore, metal oxide particles that have higher hydrophobicity and are coated with dimethylpolysiloxane have been required.
The present invention has been made in view of the above circumstances, and an object of the present invention is to provide metal oxide particles that have excellent hydrophobicity and are coated with dimethylpolysiloxane. In addition, another object of the present invention is to provide a dispersion liquid, a composition, and a cosmetic, which contain metal oxide particles coated with dimethylpolysiloxane. In addition, another object of the present invention is to provide a method for producing such metal oxide particles coated with dimethylpolysiloxane.

Solution to Problem

The present invention has the following aspects.

[1] Polysiloxane-coated metal oxide particles that are metal oxide particles with surfaces coated with a dimethylpolysiloxane, in which a hydroxyl group detection rate is 10% or less, the hydroxyl group detection rate being calculated based on an adsorption amount of a red coloring agent adsorbed on a hydroxyl group present on the surfaces of the metal oxide particles before being coated with the dimethylpolysiloxane and an adsorption amount of a red coloring

agent adsorbed on the hydroxyl group present on the surfaces of the metal oxide particles after being coated with the dimethylpolysiloxane.

[2] The polysiloxane-coated metal oxide particles according to [1], in which a content of the metal oxide particles is 80% by mass or more and 99% by mass or less.

[3] A dispersion liquid including the polysiloxane-coated metal oxide particles according to [1] or [2], and a dispersion medium.

[4] A composition including the dispersion liquid according to [3] and a resin.

[5] A cosmetic including the polysiloxane-coated metal oxide particles according to [1] or [2].

[6] A cosmetic including the dispersion liquid according to [3].

[7] A method for producing a polysiloxane-coated metal oxide particles, that is a method for producing the polysiloxane-coated metal oxide particles according to [1] or [2], the method including a surface treatment step of mixing metal oxide particles with a dimethylpolysiloxane having a kinematic viscosity of 500 $mm^2/s$ or more and 4,000 $mm^2/s$ or less at 25°C without using a solvent to coat a surface of the metal oxide particles with the dimethylpolysiloxane, and a step of subjecting the metal oxide particles coated with the dimethylpolysiloxane to a heat treatment at 100°C or higher and 380°C or lower.

[8] A method for producing a polysiloxane-coated metal oxide particles, that is a method for producing the polysiloxane-coated metal oxide particles according to [1] or [2], the method including a surface treatment step of heating and mixing metal oxide particles with a dimethylpolysiloxane having a kinematic viscosity of 500 $mm^2/s$ or more and 4,000 $mm^2/s$ or less at 25°C without using a solvent to coat a surface of the metal oxide particles with the dimethylpolysiloxane, in which a temperature in the heating and mixing of the surface treatment step is 100°C or higher and 380°C or lower.

[9] The method for producing a polysiloxane-coated metal oxide particles according to [7] or [8], in which the method may include a step of preparing a solution C1 obtained by dissolving a red coloring agent in toluene, a solution A1 obtained by mixing the metal oxide particles before coating, with the solution C1 and then removing the particles, and a solution B1 obtained by mixing the metal oxide particles after coating, which is obtained by the heat treatment or the heating and mixing, with the solution C1 and then removing the particles, a step of measuring absorbances for the three solutions at a wavelength of 545 nm, a step of obtaining each of an adsorption amount of the red coloring agent to the metal oxide particles before coating and an adsorption amount of the red coloring agent to the metal oxide particles after coating from the three measured absorbances, a step of obtaining a hydroxyl group detection rate of the metal oxide particles after coating from the obtained adsorption amount of the red coloring agent, a step of determining whether or not the obtained hydroxyl group detection rate is 10% or less, and a step of determining the metal oxide particles after coating as an acceptable product in a case where the hydroxyl group detection rate is determined to be 10% or less.

[10] The polysiloxane-coated metal oxide particles according to [1] or [2], in which the metal oxide particles are at least one selected from the group consisting of zinc oxide particles, titanium oxide particles, iron oxide particles, and cerium oxide particles.

[11] The polysiloxane-coated metal oxide particles according to [1] or [2], in which the metal oxide particles are dry particles including zinc oxide particles as the metal oxide particles.

[12] The polysiloxane-coated metal oxide particles according to [1] or [2], in which the dimethylpolysiloxane is a dimethylpolysiloxane having a kinematic viscosity of 500 $mm^2/s$ or more and 4,000 $mm^2/s$ or less at 25°C, which is represented by General Formula (6).

[Chem. 1]

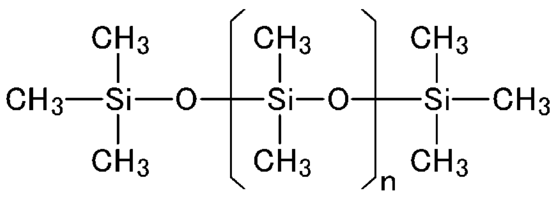

(6)

[13] The polysiloxane-coated metal oxide particles according to [1] or [2], in which the hydroxyl group detection rate is obtained by the following method: 5 ml of a solution C1 obtained by dissolving a 250 nmol red coloring agent represented by General Formula (1) in toluene, a solution A1 obtained by mixing the metal oxide particles before being coated with a dimethylpolysiloxane with the solution C1 and then removing the particles, and a solution B1 obtained by mixing the metal oxide particles after being coated with the dimethylpolysiloxane with the solution C1 and then removing the particles are prepared; absorbances of the three solutions at a wavelength of 545 nm are each

measured; an adsorption amount A3 of the red coloring agent to the metal oxide particles before being coated and an adsorption amount B3 of the red coloring agent to the metal oxide particles after being coated are each obtained from the following equation, adsorption amount = ((absorbance of solution C1 - absorbance of solution A1 or B1)/absorbance of solution C1) × 250 × $10^{-9}$ (mol)/amount (g) of metal oxide particles; and the hydroxyl group detection rate is obtained by calculation according to the following equation from the obtained adsorption amount A3 and the obtained adsorption amount B3,

$$\text{hydroxyl group detection rate (\%)} = (B3/A3) \times 100.$$

[Chem. 2]

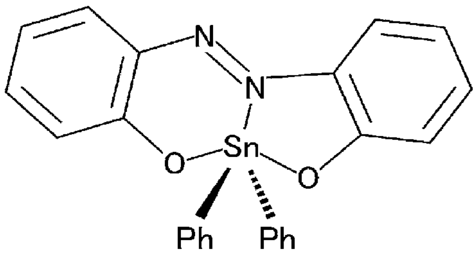

(1)

Advantageous Effects of Invention

According to the present invention, it is possible to provide a polysiloxane-coated metal oxide particle having excellent hydrophobicity. In addition, according to the present invention, it is possible to provide a dispersion liquid, a composition, and a cosmetic, which contain such polysiloxane-coated metal oxide particles. In addition, according to the present invention, it is possible to provide a method for producing such polysiloxane-coated metal oxide particles.

DESCRIPTION OF EMBODIMENTS

Embodiments of polysiloxane-coated metal oxide particles, a dispersion liquid, a composition, a cosmetic, and a method for producing polysiloxane-coated metal oxide particles according to the present invention will be described.

The present embodiments are simply specific descriptions for better understanding of the gist of the invention and do not limit the present invention unless particularly otherwise described. For example, unless otherwise specified, conditions such as materials, amounts, types, numbers, sizes, ratios, temperatures, and the like may be changed, added, or omitted as necessary. In the embodiments described below, preferred examples from each embodiment may be exchanged or shared.

First, prior to the detailed description of the present invention, an idea by the present inventors, which leads to the present invention, will be described.

As described in Patent Literature No. 1, in a case where the metal oxide particles are subjected to a surface treatment with dimethylpolysiloxane, a method of mixing dimethylpolysiloxane dissolved in a solvent such as isopropyl alcohol with zinc oxide particles and performing a heat treatment has been generally used. In addition, to sufficiently coat the surface of the metal oxide particles, a dimethylpolysiloxane having a kinematic viscosity of 50 $mm^2/s$ to 500 $mm^2/s$ at 25°C has been generally used.

To increase the hydrophobicity of the metal oxide particles, it is considered that for example, a dimethylpolysiloxane having a high kinematic viscosity is used. However, it is difficult to sufficiently coat the surface of the metal oxide particles with the dimethylpolysiloxane having a high kinematic viscosity by the method in the related art.

As a result of various studies, the present inventors have found that in a case where the metal oxide particles is mixed with a dimethylpolysiloxane having a high kinematic viscosity without using a solvent and performing treatment at a high temperature, the surface of the metal oxide particles is sufficiently coated with the dimethylpolysiloxane. That is, it has been found that dimethylpolysiloxane-coated metal oxide particles having high hydrophobicity can be obtained. In addition, by coating and heating, the dimethylpolysiloxane coats the surface of the metal oxide particles and the methyl group of the dimethylpolysiloxane is present in a state of facing outward on the surface of the metal oxide particles. Therefore, the hydrophobicity of the metal oxide particles can be increased. In the present specification, the metal oxide particles subjected to a coating treatment with a dimethylpolysiloxane may be simply abbreviated as polysiloxane-coated metal oxide particles instead of dimethylpolysiloxane-coated metal oxide particles.

More specifically, the present inventors have conducted various studies and have found the following. As the

kinematic viscosity of a dimethylpolysiloxane increases, it becomes difficult to uniformly mix the dimethylpolysiloxane with a solvent. In a case where a solvent having high hydrophobicity is used, the dimethylpolysiloxane as described above can be dissolved. However, such the solvent having high hydrophobicity is likely to remain in the polysiloxane-coated metal oxide particles, and thus it is difficult to use the solvent for cosmetics and the like.

In addition, a dimethylpolysiloxane having a low kinematic viscosity, for example, a dimethylpolysiloxane having a kinematic viscosity of about 100 $mm^2/s$ at 25°C is easily volatilized and thus it is difficult to perform treatment at a high temperature.

Then, the present inventors have studied coating metal oxide particles with a dimethylpolysiloxane having a high kinematic viscosity without using a solvent that has been considered to be essential in the related art for coating the particle surface uniformly and sufficiently. As a result, it has been found that by mixing a dimethylpolysiloxane having a kinematic viscosity within a predetermined range without using a solvent and performing a heat treatment at a high temperature, a hydroxyl group on a surface of metal oxide particles can be coated with the dimethylpolysiloxane, thereby completing the present invention.

[Polysiloxane-coated metal oxide particles]

Polysiloxane-coated metal oxide particles according to the present embodiment are metal oxide particles with surfaces coated with a dimethylpolysiloxane, in which a hydroxyl group detection rate is 10% or less, the hydroxyl group detection rate being calculated based on an adsorption amount of a red coloring agent adsorbed on a hydroxyl group present on the surfaces of the metal oxide particles before being coated with the dimethylpolysiloxane and an adsorption amount of a red coloring agent adsorbed on the hydroxyl group present on the surfaces of the metal oxide particles after being coated with the dimethylpolysiloxane. The polysiloxane-coated metal oxide particles may be dry particles.

The hydroxyl group detection rate of 10% or less may mean that the hydroxyl group on the surface of the metal oxide particles is not detected, that is, the surface of the metal oxide particles is sufficiently coated with a dimethylpolysiloxane. Therefore, the polysiloxane-coated metal oxide particles according to the present embodiment have excellent hydrophobicity.

A method for measuring a hydroxyl group detection rate in the present embodiment will be described.

In the present specification, the "hydroxyl group detection rate" is a value measured using a red coloring agent that absorbs light in the vicinity of a wavelength of 545 nm, the red coloring agent being represented by General Formula (1).

[Chem. 3]

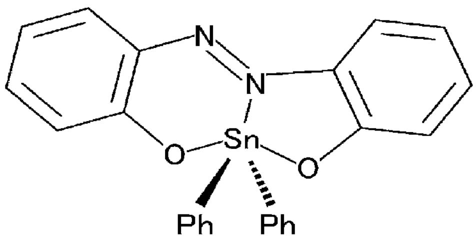

(1)

The red coloring agent represented by General Formula (1) can be produced, for example, by the following method.

1 mmol of 2,2'-dihydroxyazobenzene, 1 mmol of diphenyltin(IV) oxide as a metal source, and 30 mL of acetone are mixed with each other to prepare a mixed solution.

Next, the mixed solution is stirred at 70°C for 3 hours to perform a dehydration reaction, coordinating the diphenyltin oxide with the 2,2'-dihydroxyazobenzene.

The mixed solution after the dehydration reaction is filtered, the filtrate is collected, and the solvent is distilled off from the filtrate to obtain the red coloring agent described above.

The above-described red coloring agent is selectively adsorbed onto hydroxyl groups present on the surfaces of the metal oxide particles, and does not react with hydroxyl groups in substances such as water or alcohol. In addition, the red coloring agent does not react with dimethylpolysiloxane. Therefore, by the above-described red coloring agent, the amount of the metal hydroxyl group present on the surface of the metal oxide particles or the polysiloxane-coated metal oxide particles can qualitatively and quantitatively evaluated without being affected by moisture.

A hydroxyl group is present on the surface of the metal oxide particles. Therefore, the red coloring agent is adsorbed to the metal oxide particles before being coated with dimethylpolysiloxane. On the other hand, in the metal oxide particles coated with dimethylpolysiloxane, the hydroxyl group at the coated place is not exposed to the surface of the

polysiloxane-coated metal oxide particles. Therefore, as more places on the surface of the metal oxide particles are coated with dimethylpolysiloxane, the hydroxyl group detection rate obtained by the expression described later decreases. Therefore, the degree of coverage of the metal oxide particles with dimethylpolysiloxane can be examined by examining the adsorption amount of the red coloring agent to the metal oxide particles before coating and the adsorption amount of the red coloring agent to the metal oxide particles after coating (polysiloxane-coated metal oxide particles). That is, the smaller the hydroxyl group detection rate is, the more the surface of the metal oxide particles is coated with dimethylpolysiloxane and hydrophobized.

The hydroxyl group detection rate is preferably 8.0% or less, more preferably 7.5% or less, and still more preferably 7.0% or less. The lower limit of the hydroxyl group detection rate is 0%, but may be 0.1%, 0.5%, or 1.0%. The hydroxyl group detection rate may be 0.3% to 9.0%, 1.5% to 8.5%, 2.0% to 6.0%, 3.0% to 5.0%, or the like, as necessary.

The hydroxyl group detection rate in the present embodiment is calculated from the adsorption amounts of the red coloring agent before and after coating.

That is, the hydroxyl group detection rate of the polysiloxane-coated metal oxide particles according to the present embodiment is calculated by an expression of (B/A) $\times$ 100 in a case where the adsorption amount before the coating with the red coloring agent is defined as A and the adsorption amount after the coating with the red coloring agent is defined as B.

Specifically, the hydroxyl group detection rate (%) with the red coloring agent can be measured, for example, by the following method.

250 nmol (0.12 mg) of the red coloring agent is dissolved in toluene to 5 mL, thereby obtaining a solution C1 for evaluation of $5 \times 10^{-5}$ mol/L. An absorbance C2 of the solution C1 at a wavelength of 545 nm is measured. The absorbance can be measured using, for example, a spectrophotometer (model number: V-770, manufactured by JASCO Corporation). The unit of absorbance may be Abs.

x g of the metal oxide particles before coating is added to the solution C1 for evaluation, and the mixture is stirred and mixed at 60°C for 4 hours to prepare a mixed solution. Metal oxide particles are removed from the mixed solution by centrifugal separation, filtration, or the like to obtain a mixed solution A1 for evaluation (solution A1 after mixing). An absorbance A2 of the mixed solution A1 at a wavelength of 545 nm is measured.

y g of the polysiloxane-coated metal oxide particles to be measured is added to the above-described solution C1 for evaluation, and the mixture is stirred and mixed at 60°C for 4 hours to prepare a mixed solution. Polysiloxane-coated metal oxide particles are removed from the mixed solution by centrifugal separation, filtration, or the like to obtain a mixed solution B1 for evaluation (solution B1 after mixing). An absorbance B2 of the mixed B1 at a wavelength of 545 nm is measured.

It is noted that x and y may be adjusted by the BET specific surface area of the metal oxide particles. For example, x and y are 1 to 50 mg. In a case where the BET specific surface area is 40 $m^2/g$, it is preferable that x and y are about $4 \times 10^{-3}$ g. In a case where the BET specific surface area is 5 $m^2/g$, it is preferable that x and y are about $32 \times 10^{-3}$ g. It is preferable that x and y are the same value.

The adsorption amount (mol/g) of the red coloring agent to the metal oxide particles before coating is calculated from General Formula (2).

$$\text{Adsorption amount A3} = ((\text{C2 - A2})/\text{C2}) \times 250 \times 10^{-9}\ (\text{mol})/\text{x (g)} \ldots (2)$$

The adsorption amount (mol/g) of the red coloring agent to the polysiloxane-coated metal oxide particles is calculated from General Formula (3).

$$\text{Adsorption amount B3} = ((\text{C2 - B2})/\text{C2}) \times 250 \times 10^{-9}\ (\text{mol})/\text{y (g)} \ldots (3)$$

In the general formulae (2) and (3), since a decrease in absorbance indicates that the coloring agent is adsorbed, the adsorption amount of the above-described red coloring agent is calculated based on an idea that the decrease rate in absorbance can be converted to the adsorption rate of the coloring agent. It is noted that the General Formulae (2) and (3) can be represented by adsorption amount = ((absorbance of solution C1 - absorbance of solution A1 or B1)/absorbance of solution C1) $\times$ 250 $\times$ $10^{-9}$ (mol)/amount of metal oxide particles (g).

The hydroxyl group detection rate can be calculated by General Formula (4).

$$\text{Hydroxyl group detection rate (\%)} = (\text{B3/A3}) \times 100 \ldots (4)$$

The polysiloxane-coated metal oxide particles according to the present embodiment have a hydroxyl group detection rate of 10% or less. In a case where the hydroxyl group detection rate is 10% or less, the surface of the metal oxide particles is sufficiently coated with dimethylpolysiloxane. Therefore, the polysiloxane-coated metal oxide particles

according to the present embodiment have excellent hydrophobicity.

The content of the metal oxide particles in the total mass of the polysiloxane-coated metal oxide particles according to the present embodiment is preferably 80% by mass or more and 99% by mass or less, more preferably 85% by mass or more and 99% by mass or less, still more preferably 90% by mass or more and 99% by mass or less, and particularly preferably 95% by mass or more and 99% by mass or less. In a case where the content of the metal oxide particles is within the above-described range, the amount of the metal oxide particles added to the cosmetic or the like can be reduced to exhibit the performance inherent to the metal oxide particles. Therefore, the degree of freedom in blending design of a cosmetic or the like can be increased. The polysiloxane-coated metal oxide particles according to the present embodiment may be composed of only dimethylpolysiloxane and metal oxide particles, excluding impurities that are inevitably contained. Alternatively, the polysiloxane-coated metal oxide particles according to the present embodiment may contain a surface treatment agent other than dimethylpolysiloxane, as long as the object of the present invention is not impaired.

The BET specific surface area of the polysiloxane-coated metal oxide particles according to the present embodiment may be adjusted according to the application and is not particularly limited. For example, in a case of being used for a cosmetic, the BET specific surface area is preferably 1.5 $m^2/g$ or more and 65 $m^2/g$ or less.

In the present specification, the "BET specific surface area" means a value measured by a BET method using a fully automatic specific surface area measuring device (product name: Macsorb HM Model-1201, manufactured by Mountech Co.,Ltd.).

(BET specific surface area of polysiloxane-coated metal oxide particles)

The preferred BET specific surface area of the polysiloxane-coated metal oxide particles in a case of being used for a cosmetic will be described.

The BET specific surface area of the polysiloxane-coated metal oxide particles can be optionally selected, but is preferably 1.5 $m^2/g$ or more, more preferably 2.5 $m^2/g$ or more, and still more preferably 3.5 $m^2/g$ or more. In addition, the BET specific surface area of the polysiloxane-coated metal oxide particles is preferably 65 $m^2/g$ or less and more preferably 60 $m^2/g$ or less. For example, the BET specific surface area may be 40 $m^2/g$ or less, 30 $m^2/g$ or less, 20 $m^2/g$ or less, or 10 $m^2/g$ or less. As necessary, the BET specific surface area of the polysiloxane-coated metal oxide particles may be 8 $m^2/g$ or less or 6.0 $m^2/g$ or less. The upper limit value and lower limit value described above of the BET specific surface area of the polysiloxane-coated metal oxide particles can be arbitrarily combined.

In a case where the BET specific surface area of the polysiloxane-coated metal oxide particles is 1.5 $m^2/g$ or more and 65 $m^2/g$ or less, the transparency and the ultraviolet shielding properties are excellent in a case of being blended in a cosmetic.

In a case where the BET specific surface area of the polysiloxane-coated metal oxide particles is 1.5 $m^2/g$ or more and 8 $m^2/g$ or less, the hydrophobicity is more excellent.

It is noted that the value of the BET specific surface area of the metal oxide particles before being coated with dimethylpolysiloxane and the value of the BET specific surface area of the metal oxide particles (polysiloxane-coated metal oxide particles) after being coated with dimethylpolysiloxane do not change significantly.

(Average primary particle diameter of polysiloxane-coated metal oxide particles)

The average primary particle diameter of the polysiloxane-coated metal oxide particles according to the present embodiment is preferably 15 nm or more and more preferably 20 nm or more. The average primary particle diameter may be 50 nm or more, 80 nm or more, or 100 nm or more. From the viewpoint of increasing the hydrophobicity of the polysiloxane-coated zinc oxide particles, the average primary particle diameter is preferably 130 nm or more, more preferably 150 nm or more, and still more preferably 200 nm or more. In addition, the average primary particle diameter of the polysiloxane-coated metal oxide particles according to the present embodiment is preferably 300 nm or less, more preferably 270 nm or less, and still more preferably 250 nm or less.

In a case where the average primary particle diameter of the polysiloxane-coated metal oxide particles is 15 nm or more and 300 nm or less, the transparency and the ultraviolet shielding properties are excellent in a case of being blended in the cosmetic.

The average primary particle diameter of the polysiloxane-coated metal oxide particles can be calculated by General Formula (5) using the BET specific surface area of the polysiloxane-coated metal oxide particles.

$$\text{Average primary particle diameter (nm)} = 6{,}000/(\text{BET specific surface area } (m^2/g) \times \rho\ (g/cm^3)) \quad (5)$$

(In the formula, $\rho$ is set to 5.61 $g/cm^3$, which is the density of the metal oxide particles.)

Alternatively, the average primary particle diameter of the polysiloxane-coated metal oxide particles may be

obtained by the following method. That is, in a case where the dimethylpolysiloxane-coated metal oxide particles are observed using a transmission electron microscope (TEM) or the like, a predetermined number of polysiloxane-coated metal oxide particles, for example, 200 or 100 are selected. Then, the longest linear portion (maximum major axis) of each of these polysiloxane-coated metal oxide particles is measured, and these measured values are arithmetically averaged.

In a case where the polysiloxane-coated metal oxide particles are aggregated, the agglomerated particle diameter of the agglomerate is not measured. A predetermined number of the polysiloxane-coated metal oxide particles (primary particles) constituting the agglomerate are measured as the average primary particle diameter.

(Metal oxide particles)

The metal oxide particles in the present embodiment are not particularly limited. In a case where the polysiloxane-coated metal oxide particles according to the present embodiment are used as a refractive index adjusting agent, metal oxide particles including at least one selected from the group consisting of zinc oxide particles, titanium oxide particles, iron oxide particles, cerium oxide particles, zirconium oxide particles, aluminum oxide particles, copper oxide particles, tin oxide particles, tantalum oxide particles, niobium oxide particles, tungsten oxide particles, europium oxide particles, yttrium oxide particles, molybdenum oxide particles, indium oxide particles, antimony oxide particles, germanium oxide particles, bismuth oxide particles, hafnium oxide particles, potassium titanate particles, barium titanate particles, strontium titanate particles, potassium niobate particles, lithium niobate particles, calcium tungstate particles, antimony-doped tin oxide particles, and indium-doped tin oxide particles are suitably used.

In a case where the polysiloxane-coated metal oxide particles according to the present embodiment are used as an ultraviolet shielding material such as a cosmetic, zinc oxide particles, titanium oxide particles, iron oxide particles, and cerium oxide particles, each having ultraviolet shielding properties, can be used. It is preferable to use zinc oxide particles from the viewpoint that the ultraviolet wavelength range that can be shielded is wide.

(Zinc oxide particles)

The BET specific surface area of the zinc oxide particles in the present embodiment is preferably 1.5 $m^2/g$ or more and 65 $m^2/g$ or less. The BET specific surface area is more preferably 2.5 $m^2/g$ or more and still more preferably 4 $m^2/g$ or more. In addition, the BET specific surface area of the zinc oxide particles may be, for example, 60 $m^2/g$ or less, 55 $m^2/g$ or less, 50 $m^2/g$ or less, or 45 $m^2/g$ or less. As necessary, the specific surface area of the zinc oxide particles may be 40 $m^2/g$ or less, 30 $m^2/g$ or less, or 10 $m^2/g$ or less. The upper limit value and lower limit value of the BET specific surface area of the zinc oxide particles can be combined arbitrarily. In a case where the BET specific surface area of the zinc oxide particles is less than the above-described lower limit value, the transparency decreases in a case of being blended in a cosmetic, which is not preferable. In a case where the BET specific surface area of the zinc oxide particles exceeds the above-described upper limit value, the particles may be easily agglomerated in a case where the cosmetic contains a high concentration of zinc oxide particles coated with dimethylpolysiloxane (hereinafter, referred to as "polysiloxane-coated zinc oxide particles"), which is not preferable.

From the viewpoint of increasing the hydrophobicity of the polysiloxane-coated zinc oxide particles, the BET specific surface area of the zinc oxide particles is preferably 10.0 $m^2/g$ or less, more preferably 8.2 $m^2/g$ or less, still more preferably 7.1 $m^2/g$ or less, and even still more preferably 6.0 $m^2/g$ or less.

As the BET specific surface area of the zinc oxide particles decreases, the agglomeration between the particles is suppressed, and the portions not subjected to the surface treatment decrease. Therefore, the alcohol resistance is excellent.

The BET specific surface area of the zinc oxide particles in the present embodiment means a value measured by a BET method using a fully automatic specific surface area measuring device (product name: Macsorb HM Model-1201, manufactured by Mountech Co., Ltd.).

The average primary particle diameter of the zinc oxide particles in the present embodiment is preferably 15 nm or more and more preferably 20 nm or more. From the viewpoint of increasing the hydrophobicity of the polysiloxane-coated zinc oxide particles, the average particle diameter is preferably 130 nm or more, more preferably 150 nm or more, and still more preferably 200 nm or more. In addition, the average primary particle diameter of the zinc oxide particles according to the present embodiment is preferably 300 nm or less, more preferably 270 nm or less, and still more preferably 250 nm or less.

In a case where the average primary particle diameter of the above-described zinc oxide particles is 15 nm or more and 300 nm or less, the transparency and ultraviolet shielding properties are excellent in a case of being blended into a cosmetic.

In a case where the average primary particle diameter of the zinc oxide particles is 130 nm or more and 300 nm or less, polysiloxane-coated zinc oxide particles having excellent alcohol resistance are obtained.

The average primary particle diameter of the zinc oxide particles can be calculated by General Formula (5) using

the BET specific surface area of the zinc oxide particles, in the same manner as the BET specific surface area of the above-described polysiloxane-coated metal oxide particles.

In addition, the average primary particle diameter of the zinc oxide particles may be measured using a transmission electron microscope, similarly to the average primary particle diameter of the polysiloxane-coated metal oxide particles.

The hydrophobicity of the polysiloxane-coated metal oxide particles according to the present embodiment according to the limiting ethanol method is preferably 16% or more, more preferably 19% or more, still more preferably 22% or more, and even more preferably 35% or more.

In addition, the hydrophobicity of the polysiloxane-coated metal oxide particles according to the limiting ethanol method after being mixed with 50°C ethanol is preferably 16% or more, more preferably 19% or more, still more preferably 22% or more, and even still more preferably 35% or more.

Since the hydroxyl group detection rate of the polysiloxane-coated metal oxide particles according to the present embodiment is 10% or less, the hydrophobicity by the limiting ethanol method can be 16% or more, and even after being mixed with 50°C ethanol, high hydrophobicity can be maintained.

The evaluation method of hydrophobicity by the limiting ethanol method of the present embodiment will be described.

In the present specification, the limiting ethanol method is a method of adding polysiloxane-coated metal oxide particles to a solution obtained by mixing water and ethanol and observing whether or not the polysiloxane-coated metal oxide particles are precipitated.

In addition, the limiting ethanol method is a method of evaluating the degree of hydrophobicity of the surface of the polysiloxane-coated metal oxide particles at an ethanol ratio (mass) required for the polysiloxane-coated metal oxide particles to be precipitated by increasing the ethanol ratio in a case where the polysiloxane-coated metal oxide particles are not precipitated and increasing the water ratio in a case where the polysiloxane-coated metal oxide particles are precipitated.

As the ethanol ratio is higher, the surface of the metal oxide particles is hydrophobized with dimethylpolysiloxane, and the hydroxyl group treatment rate is higher.

In the present specification, the "polysiloxane-coated metal oxide particles after being mixed with 50°C ethanol" means polysiloxane-coated metal oxide particles obtained by mixing 5 g of the polysiloxane-coated metal oxide particles according to the present embodiment with 45 g of ethanol, stirring the mixture at 2,000 rpm for 10 minutes with a disperser in a state being heated to 50°C, separating the mixed solution into a solid and a liquid, and drying the collected solid at 40°C for 5 hours.

In the present embodiment, in a case where the zinc oxide particles are coated with dimethylpolysiloxane, the BET specific surface area of the polysiloxane-coated zinc oxide particles tends to be smaller than the BET specific surface area of the zinc oxide particles before the coating with dimethylpolysiloxane, but the BET specific surface area of the polysiloxane-coated zinc oxide particles and the BET specific surface area of the zinc oxide particles before the coating are substantially the same size. Similarly, by coating the zinc oxide particles, the average primary particle diameter of the polysiloxane-coated zinc oxide particles tends to be larger than the average primary particle diameter of the zinc oxide particles before the coating with dimethylpolysiloxane, but the average primary particle diameter of the polysiloxane-coated zinc oxide particles and the average primary particle diameter of the zinc oxide particles before the coating with dimethylpolysiloxane are substantially the same size. Here, the term "substantially the same" means that the difference in the BET specific surface area between the zinc oxide particles and the polysiloxane-coated zinc oxide particles is about 5 $m^2/g$.

In the present embodiment, from the viewpoint of improving dispersion stability in the cosmetic, it is preferable to use high-purity zinc oxide particles.

(Dimethylpolysiloxane)

The dimethylpolysiloxane in the present embodiment is represented by General Formula (6).

It is noted that another name of the dimethylpolysiloxane of the present embodiment is dimethicone.

[Chem. 4]

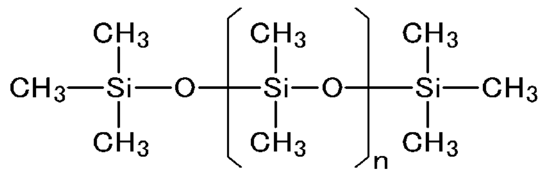

(6)

In General Formula (6), n may be appropriately selected and used as long as the hydroxyl group detection rate in the polysiloxane-coated metal oxide particles is 10% or less. For example, n is preferably 220 or more and 570 or less, more preferably 250 or more and 500 or less, and still more preferably 300 or more and 400 or less.

The dimethylpolysiloxane in the present embodiment preferably has a molecular weight of 16,000 or more and 42,000 or less, more preferably 20,000 or more and 39,000 or less, still more preferably 22,000 or more and 34,000 or less, and even still more preferably 24,000 or more and 30,000 or less.

The dimethylpolysiloxane in the present embodiment preferably has a kinematic viscosity of 500 $mm^2/s$ or more and 4,000 $mm^2/s$ or less, more preferably 700 $mm^2/s$ or more and 3,000 $mm^2/s$ or less, and still more preferably 900 $mm^2/s$ or more and 2,000 $mm^2/s$ or less at 25°C. The kinematic viscosity may be 600 $mm^2/s$ or more and 3,500 $mm^2/s$ or less, 800 $mm^2/s$ or more and 2,500 $mm^2/s$ or less, 1,000 $mm^2/s$ or more and 1,500 $mm^2/s$ or less, or the like.

In the total mass of the polysiloxane-coated metal oxide particles of the present embodiment, the content of the dimethylpolysiloxane in the present embodiment is preferably 1% by mass or more and 20% by mass or less and more preferably 1% by mass or more and 15% by mass or less. The content may be 2% by mass or more and 10% by mass or less, 3% by mass or more and 8% by mass or less, or the like.

As long as the object of the present invention is not impaired, the dimethylpolysiloxane in the present embodiment may contain a silicone oil in which a side chain or a terminal methyl group has another functional group such as a hydrogen group. However, it is preferable that the polysiloxane-coated metal oxide particles according to the present embodiment are composed of only dimethylpolysiloxane represented by General Formula (6) and metal oxide particles, excluding impurities that are inevitably contained.

In a case where the polysiloxane-coated metal oxide particles according to the present embodiment are used in a cosmetic, it is preferable that the polysiloxane-coated metal oxide particles are composed of only the dimethylpolysiloxane represented by General Formula (6) and the metal oxide particles. Since the dimethylpolysiloxane is generally used as a solvent for a cosmetic, compatibility with the cosmetic is improved. In addition, from the viewpoint of heat resistance, the above-described composition is also preferable.

It is noted that the surface treatment may be performed on the metal oxide particles using a material other than the dimethylpolysiloxane in addition to the dimethylpolysiloxane, as long as the object of the present invention is not impaired.

As the surface treatment agent other than the dimethylpolysiloxane, for example, inorganic materials such as silica and alumina, or organic materials such as a silane coupling agent, a silicone compound, a fatty acid, a fatty acid soap, a fatty acid ester, and an organic titanate compound can be used.

The polysiloxane-coated metal oxide particles according to the present embodiment are excellent in hydrophobicity since the surface of the metal oxide particles is sufficiently coated with dimethylpolysiloxane. In addition, the metal oxide particles coated with a dimethylpolysiloxane having a high molecular weight also have excellent alcohol resistance.

[Method for producing polysiloxane-coated metal oxide particles]

One embodiment of the method for producing polysiloxane-coated metal oxide particles according to the present embodiment is a method for producing polysiloxane-coated metal oxide particles according to the present embodiment, the method including a surface treatment step of mixing metal oxide particles and dimethylpolysiloxane having a kinematic viscosity of 500 $mm^2/s$ or more and 4,000 $mm^2/s$ or less at 25°C, which is represented by General Formula (6), without using a solvent, and coating a surface of the metal oxide particles with the dimethylpolysiloxane (hereinafter, referred to as a "surface treatment step"), and a step of subjecting the metal oxide particles coated with the dimethylpolysiloxane to a heat treatment at 100°C or higher and 380°C or lower (hereinafter, referred to as a "heat treatment step"). It is noted that in the heat treatment step, mixing may or may not be performed.

Here, the expression "mixing without using a solvent" in the surface treatment step means that a solvent such as alcohol added in the dry surface treatment method in the related art is not added.

As the metal oxide particles and the dimethylpolysiloxane, the same ones as described above can be used.

It is noted that the surface treatment step and the heat treatment step may be performed at the same time.

That is, another embodiment of the method for producing polysiloxane-coated metal oxide particles according to the present embodiment is a method for producing polysiloxane-coated metal oxide particles according to the present embodiment, the method including a step of heating and mixing metal oxide particles and dimethylpolysiloxane having a kinematic viscosity of 500 $mm^2/s$ or more and 4,000 $mm^2/s$ or less at 25°C, which is represented by General Formula (6), without using a solvent and coating a surface of the metal oxide particles with the dimethylpolysiloxane, in which the heating is 100°C or higher and 380°C or lower. In the heating and mixing, heating may be performed after mixing, the mixing may be performed after the heating, or the heating and the mixing may be performed at the same time. For example, in the heating and mixing, the heating may be performed while the mixing after starting the mixing, the mixing may be performed while heating after starting the heating, or the heating and the mixing may be started at the same time.

In the method for producing polysiloxane-coated metal oxide particles according to the present embodiment, no organic solvent such as an alcohol is added in the surface treatment step.

In the surface treatment step of coating with a dimethylpolysiloxane having a high kinematic viscosity, in a case where a solvent such as an alcohol is added, since the compatibility between the dimethylpolysiloxane and the alcohol or the like is low, the dimethylpolysiloxane and the alcohol or the like cannot be uniformly mixed. Therefore, in a case of performing the surface treatment with the dimethylpolysiloxane having a high kinematic viscosity, the function of the alcohol added to uniformly perform the surface treatment on the metal oxide particles cannot be sufficiently exhibited, and the surface cannot be sufficiently coated with dimethylpolysiloxane.

Therefore, in the method for producing polysiloxane-coated metal oxide particles according to the present embodiment, in a case where by the dry surface treatment method, the metal oxide particles are coated with a dimethylpolysiloxane having a predetermined kinematic viscosity and then subjecting the coated metal oxide particles to a heat treatment at a predetermined temperature without adding an alcohol or the like, such as a solvent which is essential for uniformly coating the metal oxide particles, it is possible to produce polysiloxane-coated metal oxide particles having high hydrophobicity and excellent alcohol resistance. The expression "mixed without using a solvent" does not exclude additives or catalysts that are added to the extent that the effects of the present invention are not affected, or impurities that are inevitably contained.

In the surface treatment step, while stirring the metal oxide particles as a raw material in a mixer such as a Henschel mixer or a Super mixer, the dimethylpolysiloxane is added to the metal oxide particles in the form of liquid droplets or spray, and then the metal oxide particles and the dimethylpolysiloxane are stirred at a high speed for a certain period of time.

The stirring may be performed at room temperature or 30°C to 150°C.

In a case where the surface treatment step and the heat treatment step are performed at the same time, the stirring may be performed at 100°C or higher and 380°C or lower. For example, the stirring may be performed at 100°C or higher and 350°C or lower, 110°C or higher and 330°C or lower, 130°C or higher and 300°C or lower, or 150°C or higher and 250°C or lower, as necessary.

The stirring speed is not particularly limited as long as the metal oxide particles and the dimethylpolysiloxane are mixed and the surface treatment reaction proceeds at the speed. For example, the stirring can be performed at a circumferential speed of 5 m/s to 60 m/s. Depending on the required conditions, the mixture may be stirred at a circumferential speed of 10 m/s to 40 m/s or a circumferential speed of 20 m/s to 30 m/s.

The stirring time is not particularly limited as long as the metal oxide particles and dimethylpolysiloxane are mixed and the surface treatment reaction proceeds during the time. For example, the mixture can be stirred for 1 minute to 5 hours, preferably about 30 minutes to 2 hours.

The amount of the dimethylpolysiloxane to be mixed is preferably 1.0 parts by mass or more and 20.0 parts by mass or less with respect to 100 parts by mass of the polysiloxane-coated metal oxide particles. In a case of the heating and mixing or the heat treatment at 250°C or higher, the amount of the dimethylpolysiloxane to be mixed is preferably 1.0 parts by mass or more and 10.0 parts by mass or less with respect to 100 parts by mass of the polysiloxane-coated metal oxide particles.

The amount of the dimethylpolysiloxane to be mixed is more preferably 1.1 parts by mass or more and 8.0 parts by mass or less, still more preferably 1.3 parts by mass or more and 6.5 parts by mass or less, and even still more preferably 1.5 parts by mass or more and 5.0 parts by mass or less.

In a case of the heating and mixing or the heat treatment at 100°C or higher and lower than 250°C, the amount of the dimethylpolysiloxane to be mixed is preferably 10.1 parts by mass or more and 20.0 parts by mass or less with respect to 100 parts by mass of the polysiloxane-coated metal oxide particles.

The amount of the dimethylpolysiloxane to be mixed is more preferably 10.1 parts by mass or more and 18.0 parts by mass or less, still more preferably 10.5 parts by mass or more and 15.0 parts by mass or less, and even still more preferably 11.0 parts by mass or more and 13.0 parts by mass or less.

In a case where the hydroxyl group detection rate is high, the amount of the dimethylpolysiloxane to be mixed may be increased or the heating temperature may be increased to efficiently promote the surface treatment reaction.

The heat treatment step or the heating and mixing is performed at a temperature at which the surface treatment

proceeds and the surface of the metal oxide particles is hydrophobized, and lower than a temperature at which the dimethylpolysiloxane is decomposed. That is, the treatment is performed at a temperature of 100°C to 380°C. The temperature may be 100°C to 350°C, 150°C to 320°C, 200°C to 300°C, 250°C to 280°C, or the like, as necessary. The heat treatment time may be appropriately performed for a time during which the surface is sufficiently hydrophobized. For example, the heat treatment can be performed for 30 minutes to 24 hours. The atmosphere during the heat treatment is not particularly limited as long as the surface treatment is not inhibited, and the atmosphere may be any of an air atmosphere, an oxygen atmosphere, an inert atmosphere, a reduced pressure atmosphere, or a vacuum atmosphere.

The heat treatment step may be performed while stirring.

The polysiloxane-coated metal oxide particles after the heat treatment step may be cracked with a pulverizer. The roughness of the polysiloxane-coated metal oxide particles can be suppressed by the cracking.

The cracking of the polysiloxane-coated metal oxide particles after the heat treatment can be performed, for example, using a known pulverizer. Examples of the pulverizer include atomizers, hammer mills, jet mills, impeller mills, pin mills, and the like.

According to the method for producing polysiloxane-coated metal oxide particles according to the present embodiment, it is possible to obtain polysiloxane-coated metal oxide particles having excellent hydrophobicity and excellent alcohol resistance.

[Dispersion liquid]

The dispersion liquid according to the present embodiment contains the polysiloxane-coated metal oxide particles according to the present embodiment and a dispersion medium.

The dispersion liquid according to the present embodiment also includes a highviscosity paste-like dispersion.

In a case where the dispersion liquid is used in a cosmetic, the dispersion medium can be formulated in the cosmetic and is not particularly limited as long as the polysiloxane-coated metal oxide particles can be dispersed.

As the dispersion medium, for example, water, alcohols, esters, ethers, natural oils, ester oils, silicone oils, and the like are suitably used.

Examples of the alcohols include methanol, ethanol, n-propanol, isopropanol, n-butanol, 2-butanol, octanol, glycerin, and the like.

Examples of the esters include ethyl acetate, butyl acetate, ethyl lactate, propylene glycol monomethyl ether acetate, propylene glycol monoethyl ether acetate, γ-butyrolactone, and the like.

Examples of the ethers include diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, diethylene glycol monoethyl ether, and the like.

In addition, as other dispersion media, ketones, aromatic hydrocarbons, cyclic hydrocarbons, amides, chain-like polysiloxanes, cyclic polysiloxanes, modified polysiloxanes, hydrocarbon oils, ester oils, silicone oils, higher fatty acids, higher alcohols, and the like are used.

Examples of the ketones include acetone, methyl ethyl ketone, methyl isobutyl ketone, acetyl acetone, cyclohexanone, and the like.

Examples of the aromatic hydrocarbons include benzene, toluene, xylene, ethyl benzene, and the like.

Examples of the cyclic hydrocarbons include cyclohexane and the like.

Examples of the amides include dimethylformamide, N,N-dimethylacetoacetamide, N-methylpyrrolidone, and the like.

Examples of the chain-like polysiloxanes include dimethylpolysiloxane, methylphenylpolysiloxane, diphenylpolysiloxane, and the like.

Examples of the cyclic polysiloxanes include octamethylcyclotetrasiloxane, decamethylcyclopentasiloxane, and dodecamethylcyclohexasiloxane.

Examples of the modified polysiloxanes include amino-modified polysiloxane, polyether-modified polysiloxane, alkyl-modified polysiloxane, and fluorine-modified polysiloxane.

Examples of the hydrocarbon oil include liquid paraffin, squalane, isoparaffin, branched chain-like light paraffin, petrolatum, ceresin, and the like.

Examples of the ester oil include isopropyl myristate, cetyl isooctanoate, glyceryl trioctanoate, and the like.

Examples of the silicone oil include decamethylcyclopentasiloxane, dimethylpolysiloxane, methylphenylpolysiloxane, and the like.

Examples of the higher fatty acid include lauric acid, myristic acid, palmitic acid, stearic acid, and the like.

Examples of the higher alcohol include lauryl alcohol, cetyl alcohol, stearyl alcohol, hexyl dodecanol, isostearyl alcohol, and the like.

The above-described dispersion medium may be used alone or in combination with two or more types thereof.

The dispersion liquid according to the present embodiment may contain a commonly used additive as long as the

characteristics thereof are not impaired.

As the additive, for example, a preservative, a dispersant, a dispersion aid, a stabilizer, a water-soluble binder, a viscosity improver, an oil-soluble chemical, an oil-soluble coloring agent, oil-soluble proteins, a UV absorber, or the like is suitably used.

The particle diameter (D50) of the polysiloxane-coated metal oxide particles in a case where the cumulative volume percentage of the particle size distribution in the dispersion liquid according to the present embodiment is 50% can be optionally selected, but is preferably 600 nm or less, more preferably 500 nm or less, and still more preferably 400 nm or less.

The lower limit value of the D50 in the dispersion liquid according to the present embodiment is not particularly limited, and may be, for example, 130 nm or more, 140 nm or more, or 150 nm or more. The upper limit value and lower limit value of the D50 can be arbitrarily combined.

In addition, the particle diameter (D90) of the polysiloxane-coated metal oxide particles in a case where the cumulative volume percentage of the particle size distribution in the dispersion liquid according to the present embodiment is 90% can be optionally selected, but is preferably 1 μm or less, more preferably 900 nm or less, and still more preferably 800 nm or less.

The lower limit value of the D90 in the dispersion liquid according to the present embodiment is not particularly limited, and may be, for example, 150 nm or more, 200 nm or more, or 250 nm or more. The upper limit value and lower limit value of the D90 can be arbitrarily combined.

In a case where the D50 in the dispersion liquid according to the present embodiment is 600 nm or less, the polysiloxane-coated metal oxide particles are likely to be uniformly distributed and the ultraviolet shielding effect is improved in a case where a cosmetic produced by using the dispersion liquid is applied to the skin, which is preferable. In addition, in a case where the D90 of the dispersion liquid according to the present embodiment is 1 μm or less, the transparency of the dispersion liquid is high, and the transparency of the cosmetic produced by using the dispersion liquid is also high, which is preferable.

That is, in a case where the D50 and the D90 of the dispersion liquid according to the present embodiment are within the above-described ranges, it is possible to obtain a dispersion liquid having excellent transparency and excellent ultraviolet shielding properties. In addition, the cosmetic produced using the dispersion liquid is also excellent in terms of transparency and ultraviolet shielding properties.

The cumulative volume percentage in the particle size distribution of the dispersion liquid can be measured using a dynamic light scattering particle size distribution analyzer.

The content of the polysiloxane-coated metal oxide particles in the dispersion liquid according to the present embodiment may be appropriately adjusted according to desired characteristics.

In a case where the dispersion liquid according to the present embodiment is used in a cosmetic, the content of the polysiloxane-coated metal oxide particles in the dispersion liquid can be optionally selected, but is preferably 10% by mass or more, more preferably 20% by mass or more, and still more preferably 30% by mass or more. In addition, the content of the polysiloxane-coated metal oxide particles in the dispersion liquid is preferably 90% by mass or less, more preferably 85% by mass or less, and still more preferably 80% by mass or less. The upper limit value and the lower limit value of the content of the polysiloxane-coated metal oxide particles in the dispersion liquid can be arbitrarily combined.

In a case where the content of the polysiloxane-coated metal oxide particles in the dispersion liquid is in the above-described range, the polysiloxane-coated metal oxide particles are contained at a high concentration. Therefore, a degree of freedom in formulation can be improved, and a viscosity of the dispersion liquid can be adjusted to a level which makes it easy to handle.

The viscosity of the dispersion liquid according to the present embodiment can be optionally selected, but is preferably 5 Pa·s or more, more preferably 8 Pa·s or more, still more preferably 10 Pa·s or more, and most preferably 15 Pa·s or more. In addition, the viscosity of the dispersion liquid is preferably 300 Pa·s or less, more preferably 100 Pa·s or less, still more preferably 80 Pa·s or less, and most preferably 60 Pa·s or less. The upper limit value and lower limit value of the viscosity of the dispersion liquid can be arbitrarily combined.

In a case where the viscosity of the dispersion liquid is in the above-described range, it is possible to obtain a dispersion liquid that is easy to handle even in a case where the solid content (polysiloxane-coated metal oxide particles) is contained at a high concentration.

A method for producing the dispersion liquid according to the present embodiment is not particularly limited. For example, example thereof include a method of mechanically dispersing the polysiloxane-coated metal oxide particles according to the present embodiment and a dispersion medium with a known dispersion device.

A dispersion device can be optionally selected, and examples thereof include a stirrer, a planetary mixer, a homomixer, an ultrasonic homogenizer, a sand mill, a ball mill, and a roll mill.

The dispersion liquid according to the present embodiment can be used for, in addition to the cosmetic, coating materials and the like, having an ultraviolet shielding function, a gas transmission-suppressing function, or the like.

With the dispersion liquid according to the present embodiment, since the dispersion liquid contains the

polysiloxane-coated metal oxide particles according to the present embodiment, in a case of being blended into a cosmetic, the feeling of roughness is suppressed, and transparency and ultraviolet shielding properties are excellent.

[Composition]

The composition according to the present embodiment contains the dispersion liquid according to the present embodiment and a resin.

The content of the polysiloxane-coated metal oxide particles in the composition according to the present embodiment may be appropriately adjusted according to desired characteristics. The above-described content is preferably, for example, 10% by mass or more and 40% by mass or less, and more preferably 20% by mass or more and 30% by mass or less. It is noted that the dispersion liquid according to the present embodiment and the resin are mixed with each other such that the content of the polysiloxane-coated metal oxide particles in the composition according to the present embodiment is in the above-described range.

In a case where the content of the polysiloxane-coated metal oxide particles in the composition is in the above-described range, a composition in which the characteristics of the polysiloxane-coated metal oxide particles can be sufficiently obtained since the solid content (polysiloxane-coated metal oxide particles) is contained at a high concentration, and the polysiloxane-coated metal oxide particles are uniformly dispersed, can be obtained.

The resin is not particularly limited as long as it is generally used for industrial applications, and examples thereof include an acrylic resin, an epoxy resin, a urethane resin, a polyester resin, and a silicone resin.

A content of the resin in the composition according to the present embodiment is not particularly limited, and is appropriately adjusted depending on the intended characteristics of the composition.

The composition according to the present embodiment may contain a commonly used additive as long as the characteristics thereof are not impaired.

Examples of the additive include a polymerization initiator, a dispersant, a preservative, and the like.

The method for producing the composition according to the present embodiment is not particularly limited, and examples thereof include a method of mechanically mixing the polysiloxane-coated metal oxide particles according to the present embodiment, a resin, and a dispersion medium using a known mixing device.

In addition, examples thereof include a method in which the above-described dispersion liquid and the resin are mechanically mixed with each other using a known mixing device.

Examples of the mixing device include a stirrer, a planetary mixer, a homogenizer, an ultrasonic homogenizer, and the like.

A coating film can be formed by applying the composition according to the present embodiment to an optional base material, for example, a plastic base material such as a polyester film by a normal application method such as a roll coating method, a flow coating method, a spray coating method, a screen printing method, a brush coating method, and an immersion method. These coating films can be used for various applications, such as a ultraviolet shielding film and a gas barrier film.

With the composition according to the present embodiment, since the composition contains the polysiloxane-coated metal oxide particles according to the present embodiment, the polysiloxane-coated metal oxide particles can be easily mixed with the resin, and the composition exhibits excellent transparency and ultraviolet shielding properties.

[Cosmetic]

The cosmetic according to one embodiment of the present embodiment contains at least one selected from the group consisting of the polysiloxane-coated metal oxide particles according to the present embodiment and the dispersion liquid according to the present embodiment. Alternatively, the cosmetic according to one embodiment of the present embodiment contains at least one selected from the group consisting of the polysiloxane-coated metal oxide particles according to the present embodiment, the dispersion liquid according to the present embodiment, and the composition according to the present embodiment.

The cosmetic according to another embodiment contains the cosmetic base raw material and at least one selected from the group consisting of the polysiloxane-coated metal oxide particles according to the present embodiment and the dispersion liquid according to the present embodiment. Alternatively, the cosmetic according to another embodiment contains a cosmetic base raw material, and at least one selected from the group consisting of the polysiloxane-coated metal oxide particles according to the present embodiment, the dispersion liquid according to the present embodiment, and the composition according to the present embodiment.

In the cosmetic, it is preferable to use zinc oxide particles as the metal oxide particles.

The cosmetic base raw material refers to various raw materials which form a base material of a cosmetic product. Examples of the cosmetic base raw material include an oil-based raw material, a water-based raw material, a surfactant, and a particulate raw material.

The oil-based raw material can be optionally selected, and examples thereof include oils and fats, higher fatty acids, higher alcohols, and ester oils.

The water-based raw material can be optionally selected, and examples thereof include purified water, alcohol, and a thickener.

The powder raw material can be optionally selected, and examples thereof include a colored pigment, a white pigment, a pearlescent agent, and an extender pigment.

The cosmetic according to the present embodiment can be obtained by, for example, blending the dispersion liquid according to the present embodiment into a cosmetic base raw material in the related art, such as an emulsion, a cream, a foundation, a lipstick, a blush, and an eye shadow.

The cosmetic according to the present embodiment is obtained, for example, by blending the polysiloxane-coated metal oxide particles according to the present embodiment into an oil phase or a water phase to prepare an O/W type or W/O type emulsion, and then blending the emulsion with the cosmetic base raw material.

The content of the polysiloxane-coated metal oxide particles in the cosmetic according to the present embodiment may be appropriately adjusted according to the desired characteristics. For example, the lower limit of the content of the polysiloxane-coated metal oxide particles may be 0.01% by mass or more, 0.1% by mass or more, or 1% by mass or more. In addition, the upper limit of the content of the polysiloxane-coated metal oxide particles may be 50% by mass or less, 40% by mass or less, or 30% by mass or less. The upper limit value and the lower limit value of the content of the polysiloxane-coated metal oxide particles in the cosmetic can be arbitrarily combined.

Hereinafter, a sunscreen cosmetic will be specifically described.

In the sunscreen cosmetic, to effectively shield ultraviolet rays, particularly long-wavelength ultraviolet rays (UVA), and in order to achieve a favorable feeling of use with minimal powderiness and creaking, it is also preferable to adjust the content of the polysiloxane-coated metal oxide particles. For example, the lower limit of the content of the polysiloxane-coated metal oxide particles in the sunscreen cosmetic is preferably 0.01% by mass or more, more preferably 0.1% by mass or more, and still more preferably 1% by mass or more. In addition, the upper limit of the content of the polysiloxane-coated metal oxide particles in the sunscreen cosmetic may be 50% by mass or less, 40% by mass or less, or 30% by mass or less. The upper limit value and the lower limit value of the content of the polysiloxane-coated metal oxide particles in the sunscreen cosmetic can be arbitrarily combined. In addition, within the above-described range, a preferred range can be optionally selected, such as 5% by mass to 15% by mass or 10% by mass to 20% by mass.

The sunscreen cosmetic may contain a hydrophobic dispersion medium, inorganic fine particles or inorganic pigment other than the polysiloxane-coated metal oxide particles, a hydrophilic dispersion medium, fats and oils, a surfactant, a moisturizer, a viscosity improver, a pH adjuster, a nutrient, an antioxidant, a fragrance, a preservative, a dispersant, an antifoaming agent, a coloring agent, a cosmetic ingredient, a polymeric substance, a bio-derived ingredient, a plant-derived ingredient, an antibacterial agent, a bactericide, a fungicide, an aqueous ingredient, an oily ingredient, a vitamin supplement, an emulsifier, a stabilizer, a solubilizer, a pearlescent agent, a refatting substance, or the like as necessary.

Examples of the hydrophobic dispersion medium include hydrocarbon oils, ester oils, silicone oils, higher fatty acids, and higher alcohols.

Examples of the hydrocarbon oil include liquid paraffin, squalane, isoparaffin, branched chain-like light paraffin, petrolatum, ceresin, and the like.

Examples of the ester oil include isopropyl myristate, cetyl isooctanoate, glyceryl trioctanoate, and the like.

Examples of the silicone oil include decamethylcyclopentasiloxane, dimethylpolysiloxane, methylphenylpolysiloxane, and the like.

Examples of the higher fatty acid include lauric acid, myristic acid, palmitic acid, stearic acid, and the like.

Examples of the higher alcohol include lauryl alcohol, cetyl alcohol, stearyl alcohol, hexyl dodecanol, isostearyl alcohol, and the like.

Examples of the inorganic fine particles or inorganic pigment other than the polysiloxane-coated metal oxide particles contained in the cosmetic include calcium carbonate, calcium phosphate (apatite), magnesium carbonate, calcium silicate, magnesium silicate, aluminum silicate, kaolin, talc, titanium oxide, aluminum oxide, yellow oxide of iron, γ-iron oxide, cobalt titanate, cobalt violet, and silicon oxide.

The sunscreen cosmetic may further contain at least one organic ultraviolet absorber. The cosmetic containing both the polysiloxane-coated metal oxide particles and the organic ultraviolet absorber is preferable because the cosmetic broadens the ultraviolet shielding region through a booster effect and also enhances ultraviolet shielding properties.

Examples of the organic ultraviolet absorber include a benzotriazole-based ultraviolet absorber, a benzoyl methane-based ultraviolet absorber, a benzoic acid-based ultraviolet absorber, an anthranilic acid-based ultraviolet absorber, a salicylic acid-based ultraviolet absorber, a cinnamic acid-based ultraviolet absorber, a silicone-based cinnamic acid ultraviolet absorber, a triazine-based ultraviolet absorber, and the like.

Examples of the benzotriazole-based ultraviolet absorber include 2,2'-hydroxy-5-methylphenylbenzotriazole, 2-(2'-hydroxy-5'-t-octylphenyl)benzotriazole, 2-(2'-hydroxy-5'-methylphenyl)benzotriazole, and the like.

Examples of the benzoyl methane-based ultraviolet absorber include dibenzalazine, dianisoylmethane, 4-tert-butyl-4'-methoxydibenzoylmethane, 1-(4'-isopropylphenyl)-3-phenyl propane-1,3-dione, 5-(3,3'-dimethyl-2-norbornylidene)-3-pentane-2-one, and the like.

Examples of the benzoic acid-based ultraviolet absorber include paraaminobenzoic acid (PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA methyl ester, and the like.

Examples of the anthranilic acid-based ultraviolet absorber include homomenthyl-N-acetyl anthranilate and the like.

Examples of the salicylic acid-based ultraviolet absorber include amyl salicylate, menthyl salicylate, homo menthyl salicylate, octyl salicylate, phenyl salicylate, benzyl salicylate, p-2-propanol phenyl salicylate, and the like.

Examples of the cinnamic acid-based ultraviolet absorber include octyl methoxycinnamate(ethylhexyl methoxycinnamate), di-para methoxy cinnamate-mono-2-glyceryl ethylhexanoate, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate(2-ethylhexyl-p-methoxy cinnamate), 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate, glyceryl mono-2-ethylhexanoyl-diparamethoxy cinnamate, and the like.

Examples of the silicone-based cinnamic acid ultraviolet absorber include [3-bis(trimethylsiloxy)methylsilyl-1-methylpropyl]-3,4,5-trimethoxy cinnamate, [3-bis(trimethylsiloxy)methylsilyl-3-methylpropyl]-3,4,5-trimethoxy cinnamate, [3-bis(trimethylsiloxy)methylsilylpropyl]-3,4,5-trimethoxy cinnamate, [3-bis(trimethylsiloxy)methylsilylbutyl]-3,4,5-trimethoxy cinnamate, [3-tris(trimethylsiloxy)silylbutyl]-3,4,5-trimethoxy cinnamate, [3-tris(trimethylsiloxy)silyl-1-methylpropyl]-3,4-dimethoxy cinnamate, and the like.

Examples of the triazine-based ultraviolet absorber include bisethylhexyloxyphenol methoxyphenyl triazine, ethylhexyl triazone, methylene bisbenzotriazolyl tetramethylbutylphenol, tris-biphenyl triazine, diethylhexyl butamido triazone, and the like.

Examples of the organic ultraviolet absorber other than the above-described ultraviolet absorbers include 3-(4'-methylbenzylidene)-d,l-camphor, 3-benzylidene-d,l-camphor, urocanic acid, ethyl urocanate esters, 2-phenyl-5-methylbenzoxazole, 5-(3,3'-dimethyl-2-norbornylidene)-3-pentane-2-one, silicone-modified ultraviolet absorbers, fluorine-modified ultraviolet absorbers, and the like. The above-described ultraviolet absorber may be used alone or in combination with two or more types thereof.

A critical wavelength of the cosmetic according to the present embodiment is preferably 370 nm or higher. In a case where the critical wavelength of the cosmetic is 370 nm or higher, a wide range of long-wavelength ultraviolet rays (UVA) and shortwavelength ultraviolet rays (UVB) can be shielded.

According to the cosmetic according to the present embodiment, at least one selected from the group consisting of the polysiloxane-coated metal oxide particles according to the present embodiment, the dispersion liquid according to the present embodiment, and the composition according to the present embodiment is contained. Therefore, it is possible to obtain a cosmetic having excellent quality stability.

[Examples]

Hereinafter, the present invention will be more specifically described with Examples and Comparative Examples, but the present invention is not limited to Examples.

[Example 1]

"Production of polysiloxane-coated zinc oxide particles"

98 parts by mass of zinc oxide particles A1 (BET specific surface area: 40 $m^2/g$, manufactured by Sumitomo Osaka Cement Co., Ltd.) and 2 parts by mass of dimethylpolysiloxane (product name: KF-96-1,000cs, manufactured by Shin-Etsu Chemical Co., Ltd.) were mixed at a circumferential speed of 15 m/s in a Henschel mixer heated to 60°C.

Next, the mixture was heat-treated at 350°C for 3 hours to obtain polysiloxane-coated zinc oxide particles B1 of Example 1.

(Measurement of hydroxyl group detection rate)

"Production of red coloring agent"

1 mmol of 2,2'-dihydroxyazobenzene, 1 mmol of diphenyltin(IV) oxide, and 30 mL of acetone were mixed with

each other to prepare a mixed solution.

Next, the mixed solution was stirred at 70°C for 3 hours to perform a dehydration reaction, coordinating the diphenyltin oxide with the 2,2'-dihydroxyazobenzene.

The mixed solution after the dehydration reaction was filtered, the filtrate was collected, and the solvent was distilled off from the filtrate to obtain the red coloring agent represented by General Formula (1).

"Production of solution for evaluation"

250 nmol (0.12 mg) of the obtained red coloring agent was dissolved in toluene to prepare 5 mL of a solution C1 for evaluation, having a concentration of $5 \times 10^{-5}$ mol/L. The absorbance C2 of the solution C1 for evaluation at 545 nm was measured with a spectrophotometer (model number: V-770, manufactured by JASCO Corporation).

The zinc oxide particles A1 were added to the solution C1 in an amount of 4.0 mg, and the mixture was stirred and mixed at 60°C for 4 hours to prepare a mixed solution. The mixed solution was filtered through a syringe filter (0.2 μm), and an absorbance A2 of the filtrate at 545 nm was measured.

4.0 mg of the polysiloxane-coated zinc oxide particles B1 of Example 1 was added to the solution C1, and the mixture was stirred and mixed at 60°C for 4 hours to prepare a mixed solution. The mixed solution was filtered through a syringe filter (0.2 μm), and an absorbance B2 of the filtrate at 545 nm was measured.

The adsorption amount of the red coloring agent to the zinc oxide particles and the adsorption amount of the red coloring agent to the polysiloxane-coated zinc oxide particles were calculated by General Formula (2) and General Formula (3).

$$\text{Adsorption amount B3 of coloring agent in polysiloxane-coated zinc oxide particles} = ((C2 - B2)/C2) \times 250 \times 10^{-9}\,(\text{mol})/4 \times 10^{-3}\,(\text{g}) \quad (3)$$

$$\text{Adsorption amount A3 of coloring agent in zinc oxide particles} = ((C2 - A2)/C2) \times 250 \times 10^{-9}\,(\text{mol})/4 \times 10^{-3}\,(\text{g}) \quad (2)$$

The hydroxyl group detection rate of the polysiloxane-coated zinc oxide particles B1 of Example 1 was calculated according to General Formula (4). The result is shown in Table 1.

$$\text{Hydroxyl group detection rate} = (B3/A3) \times 100 \ldots (4)$$

(Evaluation of hydrophobicity)

The hydrophobicity of the polysiloxane-coated zinc oxide particles B1 of Example 1 was evaluated by a limiting ethanol method.

As a result, it was confirmed that 10 or more particles were precipitated when the ethanol ratio was 20% by mass.

That is, the hydrophobicity of the polysiloxane-coated zinc oxide particles B1 of Example 1 was 20%.

The result is shown in Table 1.

5 g of the polysiloxane-coated zinc oxide particles B1 of Example 1 and 45 g of ethanol were mixed and stirred with a disperser at 2,000 rpm for 10 minutes in a state of being heated to 50°C.

The mixed solution after stirring was separated into a solid and a liquid, and the collected polysiloxane-coated zinc oxide particles were dried at 40°C for 5 hours.

As a result of performing hydrophobicity evaluation by the limiting ethanol method on the polysiloxane-coated zinc oxide particles after drying, the hydrophobicity was 20% by mass.

The result is shown in Table 1.

It was confirmed that the polysiloxane-coated zinc oxide particles of Example 1 had the same hydrophobicity before and after stirring in 50°C ethanol, and had excellent alcohol resistance.

[Example 2]

Polysiloxane-coated zinc oxide particles E1 of Example 2 were obtained in the same manner as in Example 1, except that zinc oxide particles D1 having a specific surface area of 5 $m^2/g$ were used instead of the zinc oxide particles A1 having the BET specific surface area of 40 $m^2/g$.

32 mg of the zinc oxide particles D1 was added to the solution C1 of Example 1, and the mixture was stirred and mixed at 60°C for 4 hours to prepare a mixed solution. The mixed solution was filtered through a syringe filter (0.2 μm), and an absorbance D2 of the filtrate at 545 nm was measured.

32 mg of the polysiloxane-coated zinc oxide particles E1 of Example 1 was added to the solution C1, and the mixture was stirred and mixed at 60°C for 4 hours to prepare a mixed solution. The mixed solution was filtered through a syringe filter (0.2 μm), and an absorbance E2 of the filtrate at 545 nm was measured.
The adsorption amount of the red coloring agent to the zinc oxide particles and the adsorption amount of the red coloring agent to the polysiloxane-coated zinc oxide particles were calculated by General Formula (2) and General Formula (3).

$$\text{Adsorption amount E3 of coloring agent of polysiloxane-coated zinc oxide particles} = ((C2 - E2)/C2) \times 250 \times 10^{-9}\ (\text{mol})/32 \times 10^{-3}\ (\text{g}) \quad (3)$$

$$\text{Adsorption amount D3 of coloring agent of zinc oxide particles} = ((C2 - D2)/C2) \times 250 \times 10^{-9}\ (\text{mol})/32 \times 10^{-3}\ (\text{g}) \quad (2)$$

The hydroxyl group detection rate of the polysiloxane-coated zinc oxide particles E1 of Example 2 was calculated according to General Formula (4). The result is shown in Table 1.

$$\text{Hydroxyl group detection rate} = E3/D3 \times 100 \ldots (4)$$

The hydrophobicity was evaluated in the same manner as in Example 1. The result is shown in Table 1.

[Example 3]

Polysiloxane-coated zinc oxide particles of Example 3 were obtained in the same manner as in Example 1, except that dimethylpolysiloxane having a kinematic viscosity of 3,000 $mm^2/s$ was used instead of dimethylpolysiloxane having a kinematic viscosity of 1,000 $mm^2/s$.
The hydroxyl group detection rate and the hydrophobicity were measured in the same manner as in Example 1. The result is shown in Table 1.

[Example 4]

94 parts by mass of zinc oxide particles A1 (BET specific surface area: 40 $m^2/g$, manufactured by Sumitomo Osaka Cement Co., Ltd.) and 6 parts by mass of dimethylpolysiloxane (product name: KF-96-1000cs, manufactured by Shin-Etsu Chemical Co., Ltd.) were mixed at a circumferential speed of 15 m/s in a Henschel mixer heated to 120°C for 5 hours, thereby obtaining polysiloxane-coated zinc oxide particles of Example 4.
The hydroxyl group detection rate and the hydrophobicity were measured in the same manner as in Example 1. The result is shown in Table 1.

[Example 5]

Polysiloxane-coated zinc oxide particles of Example 5 were obtained in the same manner as in Example 4, except that 88 parts by mass of zinc oxide particles and 12 parts by mass of dimethylpolysiloxane were used.
The hydroxyl group detection rate and the hydrophobicity were measured in the same manner as in Example 1. The result is shown in Table 1.

[Example 6]

Polysiloxane-coated zinc oxide particles of Example 5 were obtained in the same manner as in Example 4, except that 96 parts by mass of zinc oxide particles D1 having a specific surface area of 5 $m^2/g$ and 4 parts by mass of dimethylpolysiloxane were used.
The hydroxyl group detection rate and the hydrophobicity were measured in the same manner as in Example 1. The result is shown in Table 1.

[Example 7]

Polysiloxane-coated zinc oxide particles of Example 7 were obtained in the same manner as in Example 1, except that 96 parts by mass of zinc oxide particles and 4 parts by mass of dimethylpolysiloxane were used and the heating

temperature was 200°C.

The hydroxyl group detection rate and the hydrophobicity were measured in the same manner as in Example 1. The result is shown in Table 1.

[Example 8]

Polysiloxane-coated zinc oxide particles of Example 8 were obtained in the same manner as in Example 1, except that 94 parts by mass of zinc oxide particles and 6 parts by mass of dimethylpolysiloxane were used and the heating temperature was 200°C.

The hydroxyl group detection rate and the hydrophobicity were measured in the same manner as in Example 1. The result is shown in Table 1.

[Example 9]

Polysiloxane-coated zinc oxide particles of Example 9 were obtained in the same manner as in Example 1, except that 92 parts by mass of zinc oxide particles and 8 parts by mass of dimethylpolysiloxane were used and the heating temperature was 200°C.

The hydroxyl group detection rate and the hydrophobicity were measured in the same manner as in Example 1. The result is shown in Table 1.

[Comparative Example 1]

Polysiloxane-coated zinc oxide particles of Comparative Example 1 were obtained in the same manner as in Example 1, except that dimethylpolysiloxane having a kinematic viscosity of 5,000 $mm^2/s$ was used instead of dimethylpolysiloxane having a kinematic viscosity of 1,000 $mm^2/s$.

The hydroxyl group detection rate and the hydrophobicity were measured in the same manner as in Example 1. The result is shown in Table 1.

[Comparative Example 2]

Polysiloxane-coated zinc oxide particles of Comparative Example 2 were obtained in the same manner as in Example 1, except that dimethylpolysiloxane having a kinematic viscosity of 30 $mm^2/s$ was used instead of dimethylpolysiloxane having a kinematic viscosity of 1,000 $mm^2/s$ and the heating temperature was 200°C.

The hydroxyl group detection rate and the hydrophobicity were measured in the same manner as in Example 1. The result is shown in Table 1.

[Comparative Example 3]

75.7 parts by mass of zinc oxide particles (specific surface area: 40 $m^2/g$, manufactured by Sumitomo Osaka Cement Co., Ltd.), 22.7 parts by mass of isopropyl alcohol, and 1.5 parts by mass of dimethylpolysiloxane having a kinematic viscosity of 30 $mm^2/s$ were mixed at a circumferential speed of 15 m/s in a Henschel mixer heated to 60°C. Next, the mixture was heated at 200°C for 3 hours to obtain polysiloxane-coated zinc oxide particles of Comparative Example 3.

The hydroxyl group detection rate and the hydrophobicity were measured in the same manner as in Example 1. The result is shown in Table 1.

[Comparative Example 4]

75.7 parts by mass of zinc oxide particles (specific surface area: 40 $m^2/g$, manufactured by Sumitomo Osaka Cement Co., Ltd.), 22.7 parts by mass of isopropyl alcohol, and 1.5 parts by mass of dimethylpolysiloxane having a kinematic viscosity of 1,000 $mm^2/s$ were mixed at a circumferential speed of 15 m/s in a Henschel mixer heated to 60°C. Next, the mixture was heated at 350°C for 3 hours to obtain polysiloxane-coated zinc oxide particles of Comparative Example 4.

The hydroxyl group detection rate and the hydrophobicity were measured in the same manner as in Example 1. The result is shown in Table 1.

[Comparative Example 5]

Polysiloxane-coated zinc oxide particles of Comparative Example 5 were obtained in the same manner as in

Example 1, except that the heat treatment was performed at 200°C instead of 350°C.

The hydroxyl group detection rate and the hydrophobicity were measured in the same manner as in Example 1. The result is shown in Table 1.

[Comparative Example 6]

Polysiloxane-coated zinc oxide particles of Comparative Example 6 were obtained in the same manner as in Example 2, except that dimethylpolysiloxane having a kinematic viscosity of 30 $mm^2/s$ was used instead of dimethylpolysiloxane having a kinematic viscosity of 1,000 $mm^2/s$.

The hydroxyl group detection rate and the hydrophobicity were measured in the same manner as in Example 1. The result is shown in Table 1.

[Table 1]

| | Example 1 | Example 2 | Example 3 | Example 4 | Example 5 | Example 6 | Example 7 | Example 8 | Example 9 | Comparative Example 1 | Comparative Example 2 | Comparative Example 3 | Comparative Example 4 | Comparative Example 5 | Comparative Example 6 |
|---|---|---|---|---|---|---|---|---|---|---|---|---|---|---|---|
| Specific surface area [$m^2/g$] | 40 | 5 | 40 | 40 | 40 | 5 | 40 | 40 | 40 | 40 | 40 | 40 | 40 | 40 | 5 |
| Kinematic viscosity [$mm^2/s$] | 1,000 | 1,000 | 3,000 | 1,000 | 1,000 | 1,000 | 1,000 | 1,000 | 1,000 | 5,000 | 30 | 30 | 1,000 | 1,000 | 30 |
| Zinc oxide [parts by mass] | 98.0 | 98.0 | 98.0 | 94.0 | 88.0 | 96.0 | 96.0 | 94.0 | 92.0 | 98.0 | 98.0 | 75.7 | 75.7 | 98.0 | 98.0 |
| Dimethylpolysiloxane [parts by mass] | 2.0 | 2.0 | 2.0 | 6.0 | 12.0 | 4.0 | 4.0 | 6.0 | 8.0 | 2.0 | 2.0 | 1.5 | 1.5 | 2.0 | 2.0 |
| Isopropyl alcohol [parts by mass] | 0 | 0 | 0 | 0 | 0 | 0 | 0 | 0 | 0 | 0 | 0 | 22.7 | 22.7 | 0 | 0 |
| Total [parts by mass] | 100 | 100 | 100 | 100 | 100 | 100 | 100 | 100 | 100 | 100 | 100 | 100 | 100 | 100 | 100 |
| Heating temperature (°C) | 350 | 350 | 350 | 120 | 120 | 120 | 200 | 200 | 200 | 350 | 200 | 200 | 350 | 200 | 350 |
| Hydroxyl group detection rate (%) | 6.6 | 1.4 | 8.4 | 3.5 | 3.2 | 1.7 | 5.8 | 3.5 | 2.8 | 17.9 | 15.8 | 10.7 | 14.6 | 15.7 | 10.8 |
| Hydrophobicity (%) | 20 | 40 | 18 | 26 | 38 | 48 | 22 | 26 | 33 | 14 | 18 | 15 | 15 | 20 | 34 |
| Hydrophobicity after stirring at 50°C (%) | 20 | 40 | 18 | 24 | 36 | 46 | 20 | 24 | 30 | 14 | 10 | 8 | 12 | 12 | 26 |

By comparing Examples and Comparative Examples, it was confirmed that, in the case of the polysiloxane-coated zinc oxide particles obtained by subjecting zinc oxide particles to a surface treatment at a high temperature with dimethylpolysiloxane having a kinematic viscosity of 500 $mm^2/s$ or more and 4,000 $mm^2/s$ or less without using a solvent, the hydroxyl group detection rate was low. In addition, it was confirmed that in the case of the polysiloxane-coated zinc oxide particles obtained by subjecting zinc oxide particles to a surface treatment using a predetermined amount or more of dimethylpolysiloxane having a kinematic viscosity of 500 $mm^2/s$ or more and 4,000 $mm^2/s$ or less without using a solvent, the hydroxyl group detection rate was low. A low hydroxyl group detection rate indicates that the amount of hydroxyl groups present on the surface of the zinc oxide particles is small. That is, it was confirmed that the surface-treated zinc oxide particles (polysiloxane-coated zinc oxide particles) of Examples were sufficiently coated with dimethylpolysiloxane.

The dimethylpolysiloxane is more hydrophobic and more resistant to alcohol as the kinematic viscosity is higher. In the polysiloxane-coated zinc oxide particles of Examples, the surface of the zinc oxide particles can be sufficiently coated with dimethylpolysiloxane having a high kinematic viscosity by using the method for producing polysiloxane-coated metal oxide particles according to the present embodiment. Therefore, in the polysiloxane-coated zinc oxide particles of Examples, the hydroxyl group derived from the zinc oxide particles is not exposed on the surface, and thus the hydrophobicity is excellent.

In addition, dimethylpolysiloxane having a high kinematic viscosity has high hydrophobicity and excellent alcohol resistance. Accordingly, the polysiloxane-coated zinc oxide particles of Examples have high hydrophobicity and excellent alcohol resistance. Therefore, it is easy to formulate into the oil-based cosmetic, and the quality stability after the formulation into the cosmetic is excellent.

INDUSTRIAL APPLICABILITY

The present invention provides metal oxide particles which are coated with dimethylpolysiloxane and have excellent hydrophobicity. The polysiloxane-coated metal oxide particles according to the embodiment of the present invention have high hydrophobicity and excellent alcohol resistance. Therefore, the polysiloxane-coated metal oxide particles is easily mixed with various hydrophobic materials and has excellent quality stability in the hydrophobic material such as a cosmetic. Therefore, the polysiloxane-coated metal oxide particles according to the embodiment of the present invention are likely to ensure design quality in a case of being applied to a dispersion liquid, a composition, a coating material, and a cosmetic, and thus have a high industrial value.

## Claims

**1.** Polysiloxane-coated metal oxide particles that are metal oxide particles with surfaces coated with a dimethylpolysiloxane,
wherein a hydroxyl group detection rate is 10% or less, the hydroxyl group detection rate being calculated based on an adsorption amount of a red coloring agent adsorbed on a hydroxyl group present on the surfaces of the metal oxide particles before being coated with the dimethylpolysiloxane and an adsorption amount of a red coloring agent adsorbed on the hydroxyl group present on the surfaces of the metal oxide particles after being coated with the dimethylpolysiloxane.

**2.** The polysiloxane-coated metal oxide particles according to claim 1,
wherein a content of the metal oxide particles is 80% by mass or more and 99% by mass or less.

**3.** A dispersion liquid comprising:

the polysiloxane-coated metal oxide particles according to claim 1 or 2; and
a dispersion medium.

**4.** A composition comprising:

the dispersion liquid according to claim 3; and
a resin.

**5.** A cosmetic comprising:
the polysiloxane-coated metal oxide particles according to claim 1 or 2.

**6.** A cosmetic comprising:

the dispersion liquid according to claim 3.

**7.** A method for producing a polysiloxane-coated metal oxide particles, that is a method for producing the polysiloxane-coated metal oxide particles according to claim 1 or 2, the method comprising:

a surface treatment step of mixing metal oxide particles with a dimethylpolysiloxane having a kinematic viscosity of 500 $mm^2/s$ or more and 4,000 $mm^2/s$ or less at 25°C without using a solvent to coat a surface of the metal oxide particles with the dimethylpolysiloxane; and
a step of subjecting the metal oxide particles coated with the dimethylpolysiloxane to a heat treatment at 100°C or higher and 380°C or lower.

**8.** A method for producing a polysiloxane-coated metal oxide particles, that is a method for producing the polysiloxane-coated metal oxide particles according to claim 1 or 2, the method comprising:

a surface treatment step of heating and mixing metal oxide particles with a dimethylpolysiloxane having a kinematic viscosity of 500 $mm^2/s$ or more and 4,000 $mm^2/s$ or less at 25°C without using a solvent to coat a surface of the metal oxide particles with the dimethylpolysiloxane,
wherein a temperature in the heating and mixing of the surface treatment step is 100°C or higher and 380°C or lower.

**9.** The polysiloxane-coated metal oxide particles according to claim 1,
wherein the metal oxide particles are dry particles including zinc oxide particles as the metal oxide particles.

**10.** The polysiloxane-coated metal oxide particles according to claim 1,
wherein the hydroxyl group detection rate is obtained by the following method:

5 ml of a solution C1 obtained by dissolving 250 nmol of a red coloring agent represented by General Formula (1) in toluene, a solution A1 obtained by mixing the metal oxide particles before being coated with a dimethylpolysiloxane with the solution C1 and then removing the particles, and a solution B1 obtained by mixing the metal oxide particles after being coated with dimethylpolysiloxane with the solution C1 and then removing the particles are prepared;
absorbances of the three solutions at a wavelength of 545 nm are each measured;
an adsorption amount A3 of the red coloring agent to the metal oxide particles before being coated and an adsorption amount B3 of the red coloring agent to the metal oxide particles after being coated are each obtained from the following equation,

adsorption amount = ((absorbance of solution C1 - absorbance of solution A1 or B1)/absorbance of solution C1) $\times$ 250 $\times$ $10^{-9}$ (mol)/amount (g) of metal oxide particles;

and
the hydroxyl group detection rate is obtained by calculation according to the following equation from the obtained adsorption amount A3 and the obtained adsorption amount B3,

$$\text{hydroxyl group detection rate (\%)} = (B3/A3) \times 100,$$

[Chem. 1]

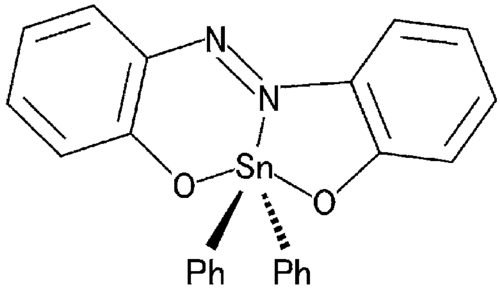

(1)

.

## INTERNATIONAL SEARCH REPORT

International application No.
**PCT/JP2023/037244**

**A. CLASSIFICATION OF SUBJECT MATTER**

***C01G 9/02***(2006.01)i; ***C09D 17/00***(2006.01)i; ***A61Q 1/00***(2006.01)i; ***A61K 8/27***(2006.01)i; ***C09C 3/10***(2006.01)i
FI: C01G9/02 A; A61K8/27; A61Q1/00; C09C3/10; C09D17/00

According to International Patent Classification (IPC) or to both national classification and IPC

**B. FIELDS SEARCHED**

Minimum documentation searched (classification system followed by classification symbols)

C01G9/02; C09D17/00; A61Q1/00; A61K8/27; C09C3/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2019/225491 A1 (KOSE CORPORATION) 28 November 2019 (2019-11-28) paragraphs [0097]-[0102] | 1-6, 9, 10 |
| A | | 7, 8 |
| X | JP 2019-178124 A (KOSE CORPORATION) 17 October 2019 (2019-10-17) paragraph [0053] | 1, 2, 5, 10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 December 2023** | **09 January 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/037244**

| Patent document cited in search report | Publication date (day/month/year) | Patent family member(s) | Publication date (day/month/year) |
|---|---|---|---|
| WO 2019/225491 A1 | 28 November 2019 | EP 3798191 A1<br>paragraphs [0152]-[0158]<br>CN 111683904 A<br>KR 10-2021-0012999 A<br>TW 202003389 A | |
| JP 2019-178124 A | 17 October 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022164568 A
- JP 2018012679 A
- JP 10155774 A
- JP 0155774 H